# EUROPEAN PATENT SPECIFICATION

(11) **EP 3 861 981 B1**
(45) Date of publication and mention of the grant of the patent: **30.08.2023**
(21) Application number: 20156098.4
(22) Date of filing: 07.02.2020
(51) Int. Cl.: A61K 9/00, A61K 9/06, A61K 31/4178, A61K 47/10, A61K 47/32, A61K 47/14, A61P 17/00, A61P 31/10

(54) **NOVEL SERTACONAZOLE COMPOSITIONS**
NEUE SERTACONAZOL-ZUSAMMENSETZUNGEN
NOUVELLES COMPOSITIONS DE SERTACONAZOLE

(43) Date of publication of application: 11.08.2021
(73) Proprietor: Dr. Pfleger Arzneimittel GmbH, 96052 Bamberg (DE)
(72) Inventor: SCHWANTES, Ulrich, 96129 Strullendorf (DE); NEUMEISTER, Claudia, 96049 Bamberg (DE); GÖTZ, Marcus Rudolf, 34399 Wesertal (DE); KRACHT, Tobias, 37073 Göttingen (DE); MÜLLER-GOYMANN, Christel C., 38104 Braunschweig (DE)
(74) Representative: Eisenführ Speiser

(56) References cited:
- WO-A1-2011/061155
- DE-A1-102009 038 512
- US-A1- 2005 181 999
- US-A1- 2006 142 304

## Description

The present invention relates to novel compositions comprising sertaconazole, one or more solvent(s), one or more solubilizer(s) and one or more gel or film former(s). The present invention further relates to such pharmaceutical composition for use in the treatment and/or prevention of nail disorders and to a kit comprising such a pharmaceutical composition.

Nail disorders as well as skin disorders, particularly fungal nail or skin disorders, are a physical as well as a mental stress for humans and usually strongly reduce the quality of life of the patients. Usually these fungal disorders appear in parallel. However, due to the structural difference of the skin and the nails, it is currently not possible to treat these disorders with the same topical medicinal product: The formulation of the antimycotic product must be designed such that the active substance therein is provided to the infected tissue in a sufficient amount, i.e. the permeation to the precise infectious site must be sufficiently high. The nails are typically hard to penetrate and thus require a high amount of active substance. In contrast, infected skin regions are more easily to penetrate and low amounts of active substance are necessary. Furthermore, systemic side-effects may occur, in case that too high amounts of active ingredient are applied to the skin.

Onychomycosis, a fungal infection of the human nail plate, is the most common nail disorder with a proportion of over 50% (Ghannoum, M. A. et al., Journal of the American Academy of Dermatology 2000, 43 (4), 641-648). Its prevalence is 10%, increasing to 20% beyond the age of 60 and to 50% above the age of 70. Onychomycosis can be caused by dermatophytes, yeasts such as *candida albicans,* or non-dermatophyte molds (Westerberg, D. P. et al., American family physician 2013, 88 (11), 762-770). The most common cause are dermatophytes, which are responsible for 90% of toenail infections (Ellis, D. H. et al., The British journal of dermatology 1997, 136 (4), 490-493). Among those, *Trichophyton rubrum* and *Trichophyton mentagrophytes* are the predominant species (Seebacher, C. et al., Mycoses 2007, 50 (4), 321-327; Gupta, A. K. et al., International journal of dermatology 1997, 36 (10), 783-787).

The current treatment options of nail disorders such as onychomycosis are often difficult: The treatment options include systemic and local administration of antimycotic drugs. Systemic treatment has the highest mycological cure rates between 48% and 78% (Gupta, A. K. et al., The British journal of dermatology 2004, 150 (3), 537-544), but has several side effects that may make it unsuitable for patients with other diseases such as heart and liver disease *(*Antifungal drugs 2009, 7 (88), 95-102, quiz 103-4). Topical treatment is an alternative with only few side effects, although the mycological cure rates are lower than in systemic treatment, e.g. with values between 29% and 36% for a ciclopirox lacquer (Gupta, A. K. et al., Journal of the American Academy of Dermatology 2000, 43 (4 Suppl), 70-80). This is due to the barrier properties of the nail plate, which behaves like a hydrophilic membrane with low permeability for most drugs (Gupchup, G. V. et al., J. Cosmet. Sci. 1999, (50), 363-385). A formulation that increases the flux of antimycotic drugs through the nail plate into the infected nail bed would thus improve the topical therapy of onychomycosis. However, it has been shown that the hydration of the human nail plate increases the permeability, e.g. for ketoconazole, likely due to a higher flexibility of the keratin matrix in a hydrated state (Gunt, H. B., et al., Eur J Pharm Sci. 2007, 32 (4-5), 254-60). Thus, the active substances may more easily enter the or pass through the keratin matrix of the nail plate.

Ketoconazole has a higher hydrophilicity, shown by its log P value of 4.2 and 4.3 (as computed by ChemAxon and XLogP3 3.0 and as such listed in the public databases drugbank https://www.drugbank.ca/ and pubchem https://pubchem.ncbi.nlm.nih.gov/ accessed 07.01.2020). Sertaconazole has a lower hydrophilicity with a log P value of 6.2 or 6.3 respectively (as computed by ChemAxon and XLogP3 3.0 and as such listed in the public databases drugbank https://www.drugbank.ca/ and pubchem https://pubchem.ncbi.nlm.nih.gov/ accessed 07.01.2020). Thus, the likelihood for sertaconazole to pass through a hydrophile barrier is lower; making it necessary to find a formulation, that carries the adequate dose to the effective location. Surprisingly sertaconazole can be formulated in the herein claimed composition to act as an effective drug product, although its physical properties stand against such an effect (even to a higher degree than ketoconazole).

It is thus desired to provide possibly much of the medical drugs into the target area, i.e. the infected skin or nail, however, at the same time, the amount must be low enough to avoid causing side-effects.

Sertaconazole is an antimycotic drug of the imidazole type and acts by inhibiting the synthesis of ergosterol. It is a broad-spectrum antifungal with activity against various dermatophytes, including *Trichophyton rubrum* and *Trichophyton mentagrophytes* (Carrillo-Muñoz et al., Antimicrobial agents and chemotherapy 2011, 55 (9), 4420-4421). It is also active against *Candida albicans* and *Cryptococcus neoformans* as well as some gram-positive bacteria (Croxtall, J. D. et al., Drugs 2009, 69 (3), 339-359). Sertaconazole is marketed as cream and spray for treatment of fungal infections of the skin caused by dermatophytes, and as vaginal ovula or cream for treatment of vaginal yeast infection with candida species. With a predicted log P value of 6.2-6.3, it is a rather lipophilic drug consequently with a low solubility in water.

Furthermore, sertaconazole is only badly resorbed after dermal application. Due to its high lipophilic characteristics, sertaconazole remains for a long time in the lipophilic regions of the skin, i.e. the stratum corneum and the stratum lucidum.

A high water content of sertaconazole-formulations thus results in a low concentration of dissolved sertaconazole.

The primary object of the present invention was thus to provide new formulations of sertaconazole, in which an amount of the low water-soluble drug sertaconazole is included, which is sufficient for treating the skin or nail disorder(s), at the same time, the permeability of the nail plate for sertaconazole shall be particularly high.

Preferably, the same formulation can be used for treating skin and nail fungal infections without the above disadvantages.

The primary object of the present is solved by a pharmaceutical composition comprising or consisting of
a) 0.1 to 2.5 wt.-%, related to the total weight of the composition, of sertaconazole or a salt thereof, preferably sertaconazole nitrate,
b) 60 to 80 wt.-%, related to the total weight of the composition, of one, two, three, four or more solvent(s), wherein the or one, two, three or four or, respectively, all solvent(s) is / are selected from the group consisting of isopropanol, ethanol, ethyl acetate and water,
c) 12.5 to 25 wt.-%, related to the total weight of the composition, of one, two, three, four or more solubilizer(s), wherein the or one, two, three, four or five or, respectively, all solubilizer(s) is/are selected from the group consisting of medium-chain triglycerides, propylene glycol, transcutol, polyglycol M350 and Macrogol 400 and
d) 5 to 12.5 wt.-%, related to the total weight of the composition, of one, two, three or more gel or film former(s), wherein the or one, two or three or, respectively, all gel or film former(s) is/are selected from the group consisting of poloxamer 407, Eudragit RS and Eudragit RL.

The term "sertaconazole" as used herein is meant to be understood such that it encompasses the substance sertaconazole itself, as well as also any possible salts thereof, such as preferably sertaconazole nitrate.

The substance "Eudragit RS" as mentioned herein has the IUPAC name "ethyl prop-2-enoate; methyl 2-methylprop-2-enoate; trimethyl-[2-(2-methylprop-2-enoyloxy)ethyl]aza-nium; chloride" and is represented by CAS-No. 33434-24-1.

The substance "Eudragit RL" as mentioned herein has the IUPAC name "*N,N*-dimethylmethanamine; 2-methylprop-2-enoic acid" and is represented by CAS-No. 51822-44-7.

As shown in Example 1, the solubility of sertaconazole alone in the solvents isopropanol, ethanol, ethyl acetate and water was tested. To increase the solubility of sertaconazole in these solvents, the solubilizers can be used (see Examples 2 and 3).

Furthermore, gel or film former(s) are typically used to provide a suitable viscosity of the pharmaceutical composition. Particularly when applied to e.g. a nail, the pharmaceutical composition should remain at (and not flow away from) the applied location for achieving a suitable effect of sertaconazole. For this purpose, gel or film former(s) can be used in combination with the solubilizers and sertaconazole (see Example 4). The pharmaceutical compositions according to the invention also perform particularly well when being dried (see Example 5), i.e. are suitable to be applied to e.g. a nail.

Moreover, the pharmaceutical compositions according to the invention are stable during storage (see Examples 8 and 10).

The pharmaceutical compositions according to the invention release sertaconazole in principle (see Example 6) and show effects on fungal growth (see Example 11). Furthermore, the pharmaceutical compositions according to the invention penetrate the nail plate barrier as shown in examples with keratin films and bovine hoof plates (see Examples 7 and 12). Interestingly, it has been found that the pharmaceutical compositions according to the invention perform much better with regard to nail plate penetration and transport of sertaconazole than other compositions (see Example 12).

In case a further substance included in the pharmaceutical composition according to the invention can be classified as a solubilizer (component c)) as well as a gel or film former (component d)) and the substance is not mentioned in the list of solubilizers or gel or film formers to be preferably selected, the substance is, for the calculation of weight percentages or weight ratios, considered as solubilizer (component c)).

Preferably, the pharmaceutical composition according to the invention comprises or consists of
a) 0.1 to 2.5 wt.-%, related to the total weight of the composition, of sertaconazole or a salt thereof, preferably sertaconazole nitrate,
b) 60 to 80 wt.-%, related to the total weight of the composition, of one, two, three, four or more solvent(s), wherein the or one, two, three or four or, respectively, all solvent(s) is / are selected from the group consisting of isopropanol, ethanol, ethyl acetate and water, wherein the total amount of the solvent(s) from the group consisting of isopropanol, ethanol, ethyl acetate and water (as far as present) in the composition is at least 60 wt.%,
c) 12.5 to 25 wt.-%, related to the total weight of the composition, of one, two, three, four or more solubilizer(s), wherein the or one, two, three, four or five or, respectively, all solubilizer(s) is/are selected from the group consisting of medium-chain triglycerides, propylene glycol, transcutol, polyglycol M350 and Macrogol 400, wherein the total amount of the solubilizer(s) from the group consisting of medium-chain triglycerides, propylene glycol, transcutol, polyglycol M350 and Macrogol 400 (as far as present) in the composition is at least 12.5 wt.%, and
d) 5 to 12.5 wt.-%, related to the total weight of the composition, of one, two, three or more gel or film former(s), wherein the or one, two or three or, respectively, all gel or film former(s) is/are selected from the group consisting of poloxamer 407, Eudragit RS and Eudragit RL, wherein the total amount of the gel or film former(s) from the group consisting of poloxamer 407, Eudragit RS and Eudragit RL (as far as present) in the composition is at least 5 wt.%.

It is also preferred that the or, respectively, all solvent(s) in component b) is/are selected from the group consisting of isopropanol, ethanol, ethyl acetate and water.

Additionally or alternatively, it is preferred that the or, respectively, all solubilizer(s) in component c) is/are selected from the group consisting of medium-chain triglycerides, propylene glycol, transcutol, polyglycol M350 and Macrogol 400.

Additionally or alternatively, it is preferred that the or, respectively, all gel or film former(s) is/are selected from the group consisting of poloxamer 407, Eudragit RS and Eudragit RL.

Preferably, component c) of the pharmaceutical composition according to the invention comprises or consists of propylene glycol.

Preferably, component c) of the pharmaceutical composition according to the invention consists of propylene glycol, transcutol, polyglykol M350 and macrogol 400.

Additionally or alternatively, it is preferred that component b) of the pharmaceutical composition according to the invention comprises or consists of ethanol and ethyl acetate.

Additionally or alternatively, it is preferred that component d) of the pharmaceutical composition according to the invention comprises or consists of Eudragit RS or Eudragit RL.

Additionally or alternatively, it is preferred that component a) of the pharmaceutical composition according to the invention is present in the range of from 1 to 2.5 wt.-%, related to the total weight of the composition,
and/or
component b) of the pharmaceutical composition according to the invention is present in the range of from 65 to 70 wt.-%, related to the total weight of the composition, preferably wherein component b) is selected from ethanol, ethyl acetate or a mixture thereof,
   and/or
component c) of the pharmaceutical composition according to the invention is present in the range of from 17.5 to 22.5 wt.-%, related to the total weight of the composition, preferably wherein component c) is selected from propylene glycol, transcutol, polyglykol M350, macrogol 400 or a mixture thereof,
   and/or
component d) of the pharmaceutical composition according to the invention is present in the range of from 7.5 to 12.5 wt.-%, related to the total weight of the composition, preferably wherein component d) is selected from Eudragit RS, Eudragit RL or a mixture thereof.

Particularly preferably, component a) of the pharmaceutical composition according to the invention is present in the range of from 1 to 2.5 wt.-%, related to the total weight of the composition,
and/or
component b) of the pharmaceutical composition according to the invention comprises or consists of ethanol and ethyl acetate and is present in the range of from 65 to 70 wt.-%, related to the total weight of the composition,
   and/or
component c) of the pharmaceutical composition according to the invention is present in the range of from 17.5 to 22.5 wt.-%, related to the total weight of the composition, and consists of propylene glycol or of propylene glycol, transcutol, polyglykol M350 and macrogol 400,
   and/or
component d) of the pharmaceutical composition according to the invention comprises or consists of Eudragit RS or Eudragit RL and is present in the range of from 7.5 to 12.5 wt.-%, related to the total weight of the composition.

Additionally or alternatively, it is also preferred that component b) of the pharmaceutical composition according to the invention comprises or consists of isopropanol and water.

Additionally or alternatively, it is preferred that component d) of the pharmaceutical composition according to the invention comprises or consists of poloxamer 407.

Additionally or alternatively, it is preferred that component c) of the pharmaceutical composition according to the invention comprises or consists of propylene glycol and medium-chain triglycerides.

Additionally or alternatively, it is preferred that component a) of the pharmaceutical composition according to the invention is present in the range of from 0.1 to 0.75 wt.-%, related to the total weight of the composition,
and/or
component b) of the pharmaceutical composition according to the invention is present in the range of from 67.5 to 77.5 wt.-%, related to the total weight of the composition, preferably wherein component b) comprises or consists of isopropanol, water or a mixture thereof,
   and/or
component c) of the pharmaceutical composition according to the invention is present in the range of from 15 to 25 wt.-%, related to the total weight of the composition, preferably wherein component c) comprises or consists of propylene glycol, medium-chain triglycerides or a mixture thereof,
   and/or
component d) of the pharmaceutical composition according to the invention is present in the range of from 5 to 10 wt.-%, related to the total weight of the composition, preferably wherein component d) comprises or consists of poloxamer 407.

Particularly preferably, component a) of the pharmaceutical composition according to the invention is present in the range of from 0.1 to 0.75 wt.-%, related to the total weight of the composition,
and/or
component b) of the pharmaceutical composition according to the invention comprises or consists of isopropanol and water and is present in the range of from 67.5 to 77.5 wt.-%, related to the total weight of the composition,
   and/or
component c) of the pharmaceutical composition according to the invention comprises or consists of propylene glycol and medium-chain triglycerides and is present in the range of from 15 to 25 wt.-%, related to the total weight of the composition,
   and/or
component d) of the pharmaceutical composition according to the invention comprises or consists of poloxamer 407 and is present in the range of from 5 to 10 wt.-%, related to the total weight of the composition.

Typically, pharmaceutical compositions according to the invention can, for example, be present in the form of solutions, suspensions, emulsions, tinctures, gels, ointments, plasters comprising a pharmaceutical composition according to the invention, sprays and lacquers.

The pharmaceutical composition according to the invention can comprise additional components. These components are usually such that are typical for pharmaceutical compositions. Such components may comprise or consist of one or more ingredients of the following groups: fillers (e.g. cellulose, calcium carbonate), plasticizers and trickling substances (e.g. talc, magnesium stearate), coatings (e.g. polyvinylacetatphtalate, hydroxypropylmethylcellulosephtalate), softeners (e.g. triethylcitrate, dibutylphtalate), substances for granulation (lactose, gelatine), retardation (e.g. poly(meth)acrylicacidmethyl/ethyl/2-trimethyl-aminoethyl ester co-polymerisates in dispersion, vinylacetate/crotonic acid co-polymerisates), compaction (e.g. micro crystalline cellulose, lactose), solvents, suspension or dispersing agents (e.g. water, ethanol), emulsifiers (e.g. cetyl alcohol, lecithin), substances for changing rheological properties (silicondioxide, sodium alginate), substances for microbial stabilisation (e.g. benzalkonium chloride, potassium sorbate), preservatives and antioxidants (e.g. DL-alphatocopherol, ascorbic acid), substances for changing the pH value (lactic acid, citric acid), propellants and inert gases (e.g. fluorinated chlorinated hydrocarbons, carbon dioxide) dyes (iron oxides, titanium dioxide), ointment raw materials (e.g. paraffins, beeswax), and others as they appear in technical literature (e.g. Schmidt, P. C. et al., Wirk- und und Hilfsstoffe für Rezeptur, Defektur und Großherstellung 1999**,** or Bauer, K. H. et al., Lehrbuch der Pharmazeutischen Technologie 2006, 8. Auflage).

The present invention further relates to a pharmaceutical composition according to the invention, for use in the treatment and/or prevention of nail disorders.

Typically, the prevention and/or treatment comprises or consists of the step of applying a pharmaceutical composition according to the invention onto the nail to be treated and/or to the surrounding tissue.

Preferably, the nail disorder is nail mycosis, distal subungual onychomycosis, proximal subungual onychomycosis, white superficial onychomycosis, dystrophic onychomycosis and/or Candida-onychomycosis.

The invention further relates to a medical kit, comprising a pharmaceutical composition according to the invention and one or more additional components, preferably selected from the group consisting of files, application brushes, pipettes, containers, dispensers, pens, pads, tapes, foils, stickers, wraps, lamps, and polishes.
Figure 1 shows the relative sertaconazole nitrate concentration in wt.-% with regard to the content measured after preparation of the respective formulation, comparing different storage conditions (RT: room temperature; d14, d30, d90: day 14, day 30, day 90).
Figure 2 shows the relative sertaconazole nitrate concentration in wt.-% with regard to the content measured after preparation of the respective formulation, over a storage time of up to 24 weeks at 30 °C.
Figure 3A shows the droplet size in µm (determined microscopically with n=2; analysing at least 1000 particles per sample) of various formulations after 1 week of storage. Lower column: particle sizes d₁₀ to d₅₀; upper column: particle sizes d₅₀ to d₉₀.
Figure 3B shows the droplet size in µm (determined microscopically with n=2; analysing at least 1000 particles per sample) of the tested formulations after 24 week of storage. *: complete coalescence, no droplet size could be determined. Lower column: particle sizes d₁₀ to d₅₀; upper column: particle sizes d₅₀ to d₉₀.
Figure 4 shows the calculated permeability coefficient of the tested formulations through keratin films in 10⁻⁷ cm / s with n=4-11.
Figure 5 shows the flux of sertaconazole nitrate through keratin films in 10⁻⁹ g / cm² * s with n=4-11.

Preferred embodiments and further aspects of the present invention also emerge from the attached patent claims and the following examples, wherein the present invention is not limited to these examples.

### Examples:

### Example 1: Solubility of sertaconazole nitrate in different solvents

Mixtures of 2 wt.-% sertaconazole nitrate and 98 wt.-% of solvents were prepared and shaken for 24 h at 150 rpm and at room temperature. Subsequently, the samples were macroscopically examined by evaluating the amount and appearance of the sediment.

| **Solvent** | **solubility** | **sediment** | **appearance** |
|---|---|---|---|
| Water | Increasing from water to ethanol | Decreasing from water to etanol | Not dissolved, powdery, white sediment |
| Butyl acetate | | | |
| Ethyl acetate | | | |
| Isopropanol | | | |
| Ethanol | | | Low, crystalline sediment |

The solubility of sertaconazole nitrate increased from water to ethanol. Particularly ethyl acetate, isopropanol and ethanol were preferred solvents.

### Example 2: Solubility of sertaconazole nitrate in different solubilizers

Mixtures of 2 wt.-% sertaconazole nitrate and 98 wt.-% of solubilizers were prepared and shaken for 24 h at 150 rpm and at room temperature. Subsequently, the samples were macroscopically examined by evaluating the amount and appearance of the sediment.

| **Solubilizer** | **solubility** | **appearance** |
|---|---|---|
| Dimethylphthalate | Increasing from dimethylphthalate to macrogol 400 | Milky mixture with sediment |
| Glycerine | | Clear solution with sediment |
| Marcogol 300 | | |
| Polyglycol M350 | | Dissolved |
| Propylene glycol | | |
| Transcutol | | |
| Macrogol 400 | | |

The solubility of sertaconazole nitrate increased from dimethylphthalate to macrogol 400. Particularly polyglycol M350, propylene glycol, transcutol and macrogol 400 were the preferred solubilizers.

### Example 3: Solubility of sertaconazole nitrate in mixtures of different solvents and solubilizers

Mixtures of 2 wt.-% sertaconazole nitrate and 98 wt.-% of a mixture of solvent and solubilizers (the amount of solubilizer with respect to the total mixture of sertaconazole nitrate, solvent and solubilizer is indicated in the subsequent table) were prepared and shaken for 24 h at 150 rpm and at room temperature. Subsequently, the samples were macroscopically examined by evaluating the amount and appearance of the sediment.

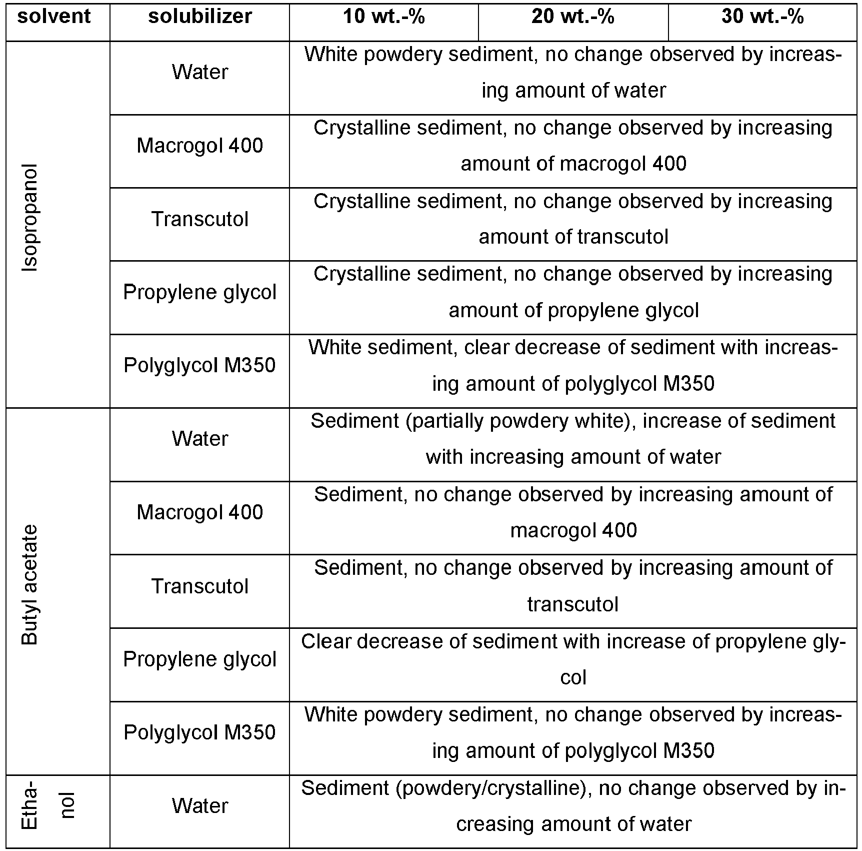

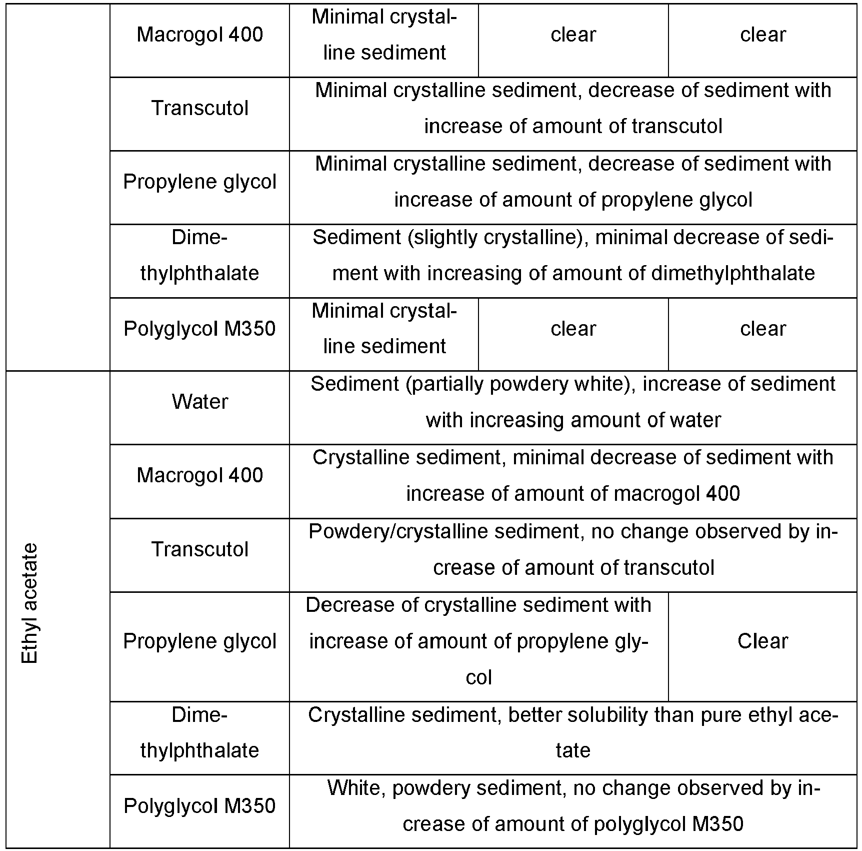

The following combinations are preferred:
Isopropanol + propylene glycol
Ethanol + 30 wt.-% macrogol 400
Ethanol + 20 wt.-% polyglycol M350
Ethyl acetate + 30 wt.-% propylene glycol

### Example 4: Solubility of gel and film former(s)

Mixtures of 10 wt.-% of the respective gel or film former and 90 wt.-% of ethanol or ethyl acetate were prepared and shaken for 24 h at 150 rpm and at room temperature. Subsequently, the samples were macroscopically examined by evaluating the amount and appearance of the sediment.

| **Gel or film former** | **Ethanol** | **Ethyl acetate** |
|---|---|---|
| Eudragit E | Clear solution, slightly yellow colour | Clear solution |
| Eudragit RL | Clear solution | Clear solution |
| Eudragit RS | Clear solution | Clear solution |
| Eudragit L 100 | Clear solution | Clear sediment |
| Eudragit L 100-55 | Clear solution | Not soluble, precipitated |
| Eudragit S 100 | Cloudy, slight sediment | Sediment, not possible to mix |
| Eudragit NM30D | Clear solution | sediment |
| Plastoid B | Sediment, "solid" | Clear solution |

Eudragit S 100 was not further investigated due to insufficient solubility.

In a next step, mixtures of 2 wt.-% sertaconazole nitrate, 10 wt.-% gel or film former, 20 wt.-% solubilizer and 68 wt.-% solvent were prepared. Mixtures with 88 wt.-% solvent and no solubilizer were also prepared as a control. The samples were placed for 24 h at room temperature while stirring and the macroscopic appearance was evaluated subsequently. The sediment was evaluated from 1 "very good" (no/little sediment) to 3 "medium" or 4 "insufficient".

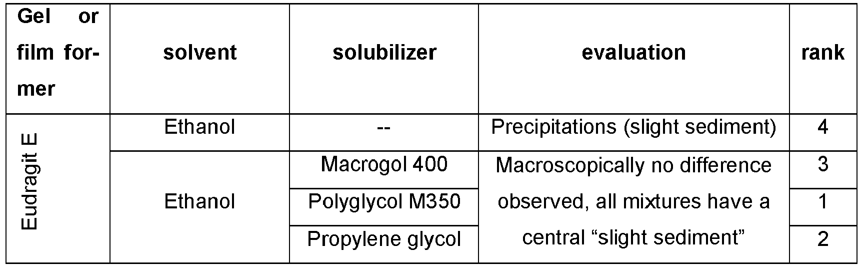

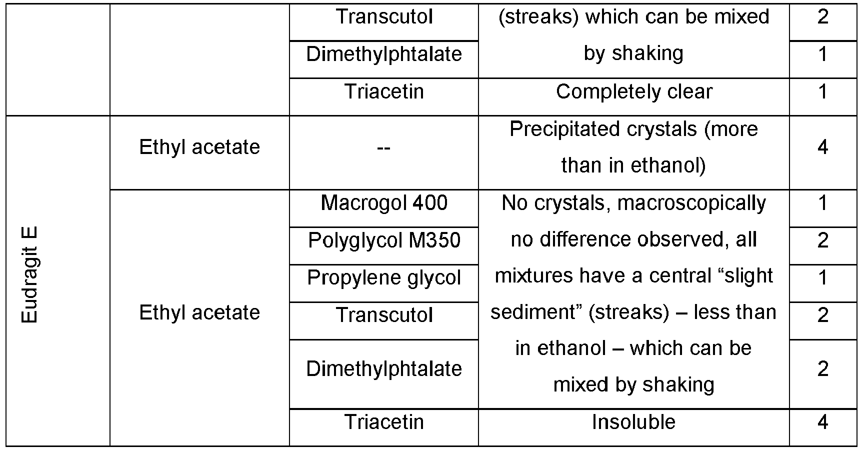

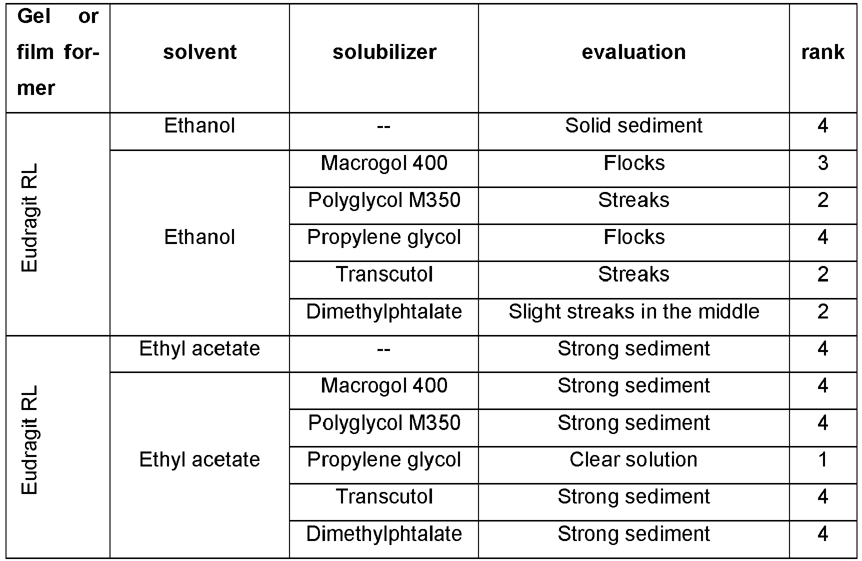

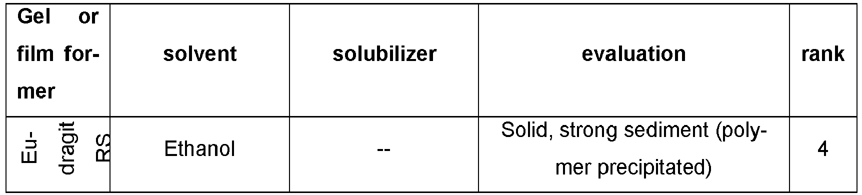

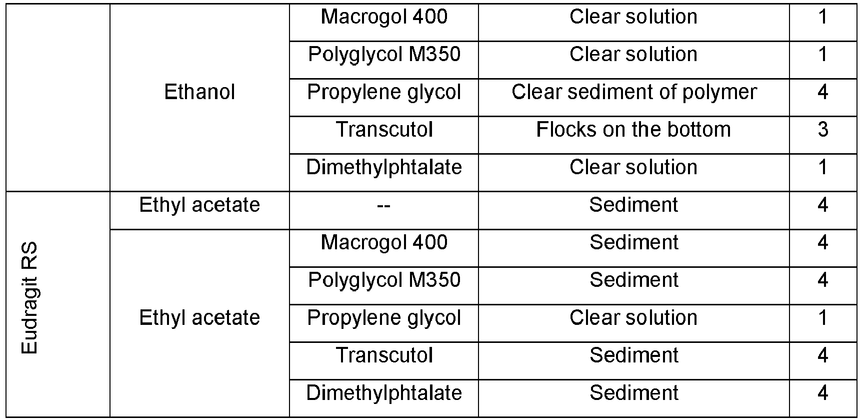

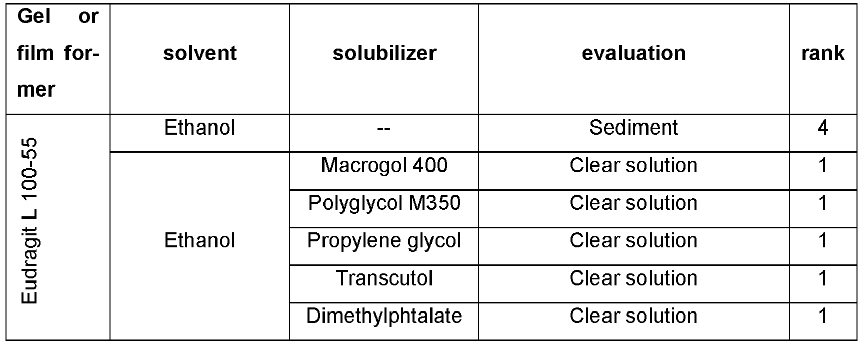

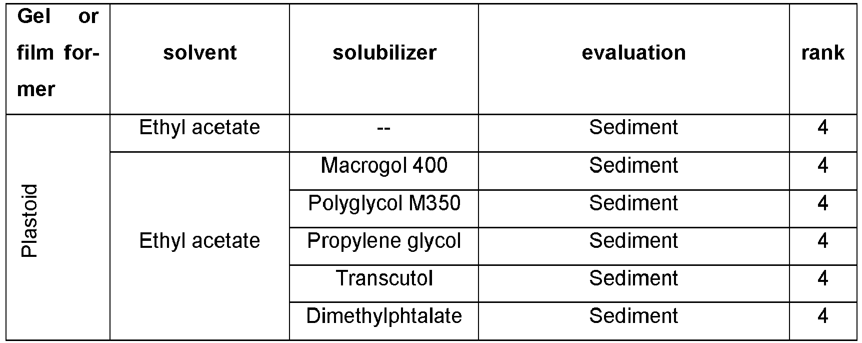

With regard to Eudragit E, ethyl acetate was proven to be the better solvent.

Eudragit RL performed worse than Eudragit E, only Eudragit RL in ethyl acetate and with 20 wt.-% propylene glycol did not show any streaks or flocks after 24 h.

With regard to Eudragit RS, several combinations showed acceptable results. When ethyl acetate was used, only propylene glycol showed acceptable results.

Eudragit L 100-55 showed good results for all examined solubilizers.

Plastoid did not show any acceptable results.

### Example 5: Drying behaviour

The most preferred combinations of Example 4 were evaluated for their drying behaviour.

One drop of the mixture as indicated in the below table (10 µl) was pipetted on a glass slide. The "stickiness" of the mixtures was analysed after 3 min and 5 min.

Mixtures with 10 wt.-% Eudragit L 100-55 and 2 wt.-% sertaconazole nitrate:

| **Mixture** | **Recipe [wt.-%]** | | | | | | **Macroscopic appearance** | **drying** | | |
|---|---|---|---|---|---|---|---|---|---|---|
| | **EtOH** | **EtAc** | **PG** | **TC** | **PG M350** | **PEG 400** | | **3 min** | **5 min** | **24 h** |
| V1 | 68 | - | 20 | - | - | - | Clear solution | - | - | + |
| V1.1 | 68 | - | 15 | 5 | - | - | Clear solution, solid after 2 h | - | - | |
| V1.2 | 68 | - | 11 | 5 | 2 | 2 | Clear solution, still flexible | - | - | |
| V1A | 34 | 34 | 20 | - | - | - | Solubility decreases from V1A to V1A.2, crystals in V1A.1 and V1A.2 | - | - | Sticky |
| V1A.1 | 34 | 34 | 15 | 5 | - | - | | - | - | Sticky |
| V1A.2 | 34 | 34 | 11 | 5 | 2 | 2 | | - | - | sticky |

| | | | | | | | | | | |
|---|---|---|---|---|---|---|---|---|---|---|
| EtOH: ethanol; EtAc: ethyl acetate; PG: propylene glycol; TC: transcutol; PG M350: polyglycol M350; PEG 400: macrogol 400; drying: "-": not dry; "+": dry | | | | | | | | | | |

Mixtures with 10 wt.-% Eudragit RS and 2 wt.-% sertaconazole nitrate:

| **Mixture** | **Recipe [wt.-%]** | | | | | | **Macroscopic appearance** | **drying** | | |
|---|---|---|---|---|---|---|---|---|---|---|
| | **EtOH** | **EtAc** | **PG** | **TC** | **PG M350** | **PEG 400** | | **3 min** | **5 min** | **24 h** |
| V2 | - | 68 | 20 | - | - | - | Clear solution | + | + | + |
| V2.1 | - | 68 | 15 | 5 | - | - | Clear solution | + | + | |
| V2.2 | - | 68 | 11 | 5 | 2 | 2 | Clear solution after preparation, after 24 precipitated | void | | |
| V2A | 34 | 34 | 20 | - | - | - | Clear solution | + | + | + |
| V2A.1 | 34 | 34 | 15 | 5 | - | - | Clear solution | + | + | + |
| V2A.2 | 34 | 34 | 11 | 5 | 2 | 2 | Clear solution | + | + | + |

| | | | | | | | | | | |
|---|---|---|---|---|---|---|---|---|---|---|
| EtOH: ethanol; EtAc: ethyl acetate; PG: propylene glycol; TC: transcutol; PG M350: polyglycol M350; PEG 400: macrogol 400; drying: "+": dry | | | | | | | | | | |

Mixtures V2A and V2A.2 showed the best results.

Mixtures with 10 wt.-% Eudragit RL and 2 wt.-% sertaconazole nitrate:

| **Mixture** | **Recipe [wt.-%]** | | | | | | **Macroscopic appearance** | **drying** | | |
|---|---|---|---|---|---|---|---|---|---|---|
| | **EtOH** | **EtAc** | **PG** | **TC** | **PG M350** | **PEG 400** | | **3 min** | **5 min** | **24 h** |
| V3 | - | 68 | 20 | - | - | - | Clear solution | + | + | + |
| V3.1 | - | 68 | 15 | 5 | - | - | Slightly opalescent, solid after 2h | - | - | |
| V3.2 | - | 68 | 11 | 5 | 2 | 2 | Clear solution, still flexible after 2 h | - | - | |
| V3A | 34 | 34 | 20 | - | - | - | Nicely clear solution | + | + | + |

| | | | | | | | | | | |
|---|---|---|---|---|---|---|---|---|---|---|
| EtOH: ethanol; EtAc: ethyl acetate; PG: propylene glycol; TC: transcutol; PG M350: polyglycol M350; PEG 400: macrogol 400; drying: "-": not dry; "+": dry | | | | | | | | | | |

Mixture V3 showed the best result.

Mixtures with 10 wt.-% Eudragit E and 2 wt.-% sertaconazole nitrate:

| **Mixture** | **Recipe [wt.-%]** | | | | | | **Macroscopic appearance** | **drying** | | |
|---|---|---|---|---|---|---|---|---|---|---|
| | **EtOH** | **EtAc** | **PG** | **TC** | **PG M350** | **PEG 400** | | **3 min** | **5 min** | **24 h** |
| V4 | - | 68 | 20 | - | - | - | Clear solution | - | + | + |
| V4.1 | - | 68 | 15 | 5 | - | - | Slightly opalescent, solid after 2h | - | - | |
| V4.2 | - | 68 | 11 | 5 | 2 | 2 | Clear solution, still flexible after 2 h | - | - | |
| V4A | 34 | 34 | 20 | - | - | - | - | | | |
| V4A.1 | 34 | 34 | 15 | 5 | - | - | - | | | |

| | | | | | | | | | | |
|---|---|---|---|---|---|---|---|---|---|---|
| EtOH: ethanol; EtAc: ethyl acetate; PG: propylene glycol; TC: transcutol; PG M350: polyglycol M350; PEG 400: macrogol 400; drying: "-": not dry; "+": dry | | | | | | | | | | |

### Example 6: sertaconazole nitrate release

Before testing the penetration and permeation of the compositions, it was examined whether the used gel or film formers release sertaconazole nitrate as such a release is essential for the transport of sertaconazole nitrate over a biological barrier such as the nail plate.

Thus, 50 µl of the recipes V1, V2, V3 or V4 were added on a glass slide. The wetted area was 6.25 cm². The glass slides were each put in water for 12 h (75 ml) and subsequently, a sample was taken and analysed via HPLC. For each recipe, triplicates were measured.

| **Recipe** | **sertaconazole nitrate release in wt.-%; [mean ± standard deviation]** |
|---|---|
| V1 | 0.6 ± 0.0 |
| V2 | 16.9 ± 1.1 |
| V3 | 13.6 ± 0.4 |
| V4 | 10.0 ± 0.2 |

As sertaconazole nitrate is only poorly released in recipe V1 (Eudragit L 100-55) and due to the poor results of the drying behaviour (Example 5) of recipe V4, only the recipes V2 to V3A were further analysed (see below).

### Example 7: Penetration and permeation

For analysing the penetration and permeation of sertaconazole nitrate, a modified diffusion cell (nail permeation apparatus) was used. The nail permeation apparatus is constructed according to a vertical diffusion cell. The nail is tightened between the acceptor chamber and the donor chamber via a PTFE-seal. The acceptor chamber and the donor chamber are made of an stainless and acid-resistant chromium-nickel-steel (1.4305). If necessary, the donor chamber may be sealed with a screw cap. The effective permeation surface in the assay was 0.785 cm². The acceptor chamber is filled with a hydrogel (nail bed analogue) to ensure a defined hydratation of the nail or, respectively, hoof membrane. The thickness of the used horse hoof membranes was in average 549 µm ± 39 µm. As nail bed analogue, a carbomer gel (Tego^{®} Carbomer 140) with 0.5 % Igepal^{®}-H₂O was used. Ige-pal^{®} serves as a solubilizer. The carbomer gel is filled into the acceptor chamber and the hoof membrane is placed thereon (in contact with the carbomer gel). Each, 100 µl of the compositions to be analysed were applied and the donor chamber was not sealed. Subsequently, an incubation of the samples was performed for 120 h and at 32.5 °C. After 120 h, the supernatant or, respectively, residues of the compositions were removed from the hoof membrane and the amount of sertaconazole nitrate was measured in the hoof membrane and the carbomer gel via extraction and HPLC. For each composition, five samples were tested.

| **Recipe** | **sertaconazole nitrate-incorporation in µg (mean ± standard deviation)** | | |
|---|---|---|---|
| | **Hoof membrane** | **Carbomer gel** | **total** |
| V2A.2 | 67.9 ± 9.2 | 5.0 ± 3.5 | 73.0 ± 10.8 |
| V2A | 62.8 ± 10.7 | 2.7 ± 0.7 | 65.5 ± 10.9 |
| V3A | 57.5 ± 12.0 | 5.2 ± 1.2 | 62.7 ± 12.3 |

The results show that sertaconazole nitrate was transported successfully over the horse hoof membrane. After 120 h, 62.7 µg (V3A), 65.5 µg (V2A) or, respectively, 73.0 µg (V2A.2) sertaconazole nitrate were transported into the horse hoof membrane and the carbomer gel underneath.

### Example 8: Storage

The stability of the three preferred mixtures (V2A, V2A.2 and V3A) was measured over three months. The mixtures were freshly prepared and the total amount of sertaconazole nitrate was measured in each mixture via HPLC (starting value). Subsequently, triplicates of the mixtures were stored at room temperature or at 40 °C (humidity: 75 %) and analysed at day 14 and after one month and after 3 months.

| **Recipe** | **Storage conditions** | **Time** | **sertaconazole nitrate amount in wt.-% of starting value** | | | |
|---|---|---|---|---|---|---|
| | | | **n = 1** | **n = 2** | **n = 3** | **Mean ± standard deviation** |
| V2A.2 | Room temperature | Day 14 | 107.4 | 94.9 | 98.9 | 100.4 ±5.2 |
| | | Month 1 | 101.9 | 103.8 | 97.4 | 101.0 ±2.7 |
| | | Month 3 | 113.4 | 93.0 | 94.2 | 100.2 ± 9.4 |
| | 40 °C (humidity: 75%) | Day 14 | 104.3 | 96.9 | 101.0 | 100.7 ±3.0 |
| | | Month 1 | 104.5 | 101.1 | 98.1 | 101.2 ± 2.6 |
| | | Month 3 | 96.5 | 97.0 | 99.1 | 97.6 ± 1.1 |
| V2A | Room temperature | Day14 | 101.0 | 98.9 | 96.6 | 98.8 ± 1.8 |
| | | Month 1 | 131.8 | 104.0 | 106.2 | 114.0 ± 12.6 |
| | | Month 3 | 92.0 | # | 94.1 | 93.1 ± 1.1 |
| | 40 °C (humidity: 75%) | Day14 | 92.9 | 115.8 | 103.0 | 103.9 ± 9.4 |
| | | Month 1 | 96.8 | 107.8 | 101.6 | 102.1 ± 4.5 |
| | | Month 3 | 100.5 | 100.2 | 97.6 | 99.5 ± 1.3 |
| V3A | Room temperature | Day14 | 101.3 | 100.7 | 92.8 | 98.3 ± 3.9 |
| | | Month 1 | 104.7 | 103.0 | 99.1 | 102.3 ±2.3 |
| | | Month 3 | 89.2 | 86.2 | 90.1 | 88.5 ± 1.7 |
| | 40 °C (humidity: 75%) | Day14 | 105.9 | 102.8 | 103.5 | 104.1 ±1.3 |
| | | Month 1 | 101.8 | 114.9 | 95.2 | 104.0 t 8.2 |
| | | Month 3 | 96.8 | 93.6 | 98.0 | 96.1 ± 1.9 |

| | | | | | | |
|---|---|---|---|---|---|---|
| #: no measurement was possible here | | | | | | |

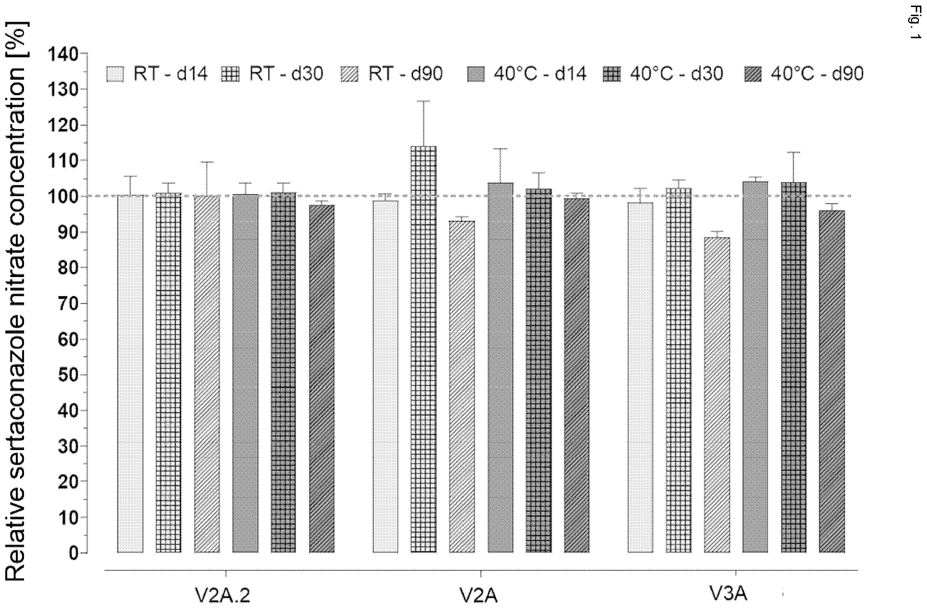

No indication of instabilities of sertaconazole nitrate in the compositions was observed over the period of 3 months (see also Fig. 1). The variations in the ranges (as well as the amounts exceeding 100 wt.-%) may be explained by the high contents of solvents and that the containers were not absolutely sealed. Thus, vaporization of the solvent may be possible, especially for recipe V2A with 68 wt.-% of ethyl acetate.

### Example 9: Stability studies

The following samples were prepared and stored for 24 weeks in sealed 2ml vials at 30 °C. Their appearance was evaluated after preparation (initial appearance) and after 24 weeks of storage.

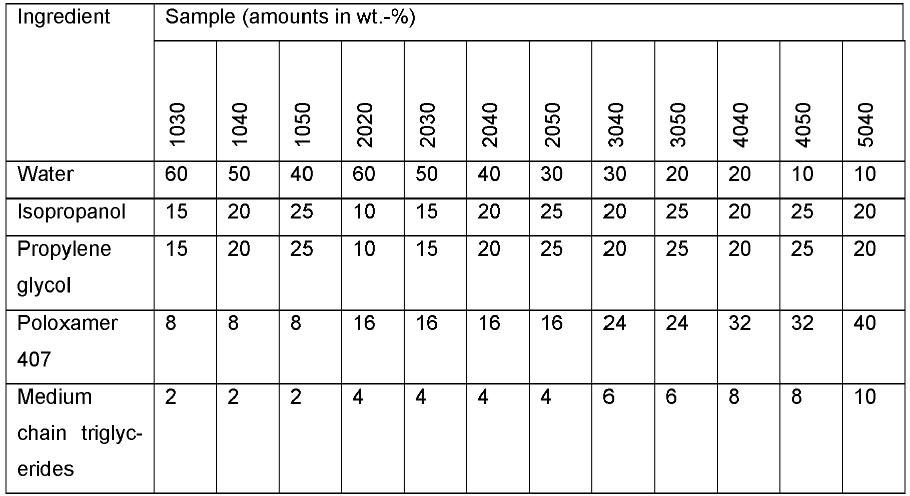

| Sample | Initial appearance | Appearance after 24 weeks |
|---|---|---|
| 1030 | Liquid, creaming | Liquid, creaming |
| 1040 | Liquid, creaming | Liquid, creaming |
| 1050 | Liquid, creaming | Liquid, coalescence |
| 2020 | Semisolid, homogenous | Semisolid, homogenous |
| 2030 | Liquid, creaming | Liquid, creaming |
| 2040 | Liquid, creaming | Liquid, creaming |
| 2050 | Liquid, creaming | Liquid, coalescence |
| 3040 | Liquid, creaming | Liquid, creaming |
| 3050 | Liquid, creaming | Liquid, coalescence |
| 4040 | Viscous, creaming | Viscous, creaming |
| 4050 | Liquid, creaming | Liquid, coalescence |
| 5040 | Viscous, creaming | Liquid, creaming |

Formulations with 50% or more of isopropanol and propylene glycol together show complete coalescence after storage. The consistencies of the tested formulations did not change over the period of 24 weeks. 40% or less of isopropanol and propylene glycol together leads to creaming within one week after manufacture, complete coalescence did not occur. The separated phases could be homogenized again by shaking the vial. Thus, the formulations with 40% or less of isopropanol and propylene glycol together were preferred. Sample 2020 was semisolid after preparation and was thus not excluded from further studies.

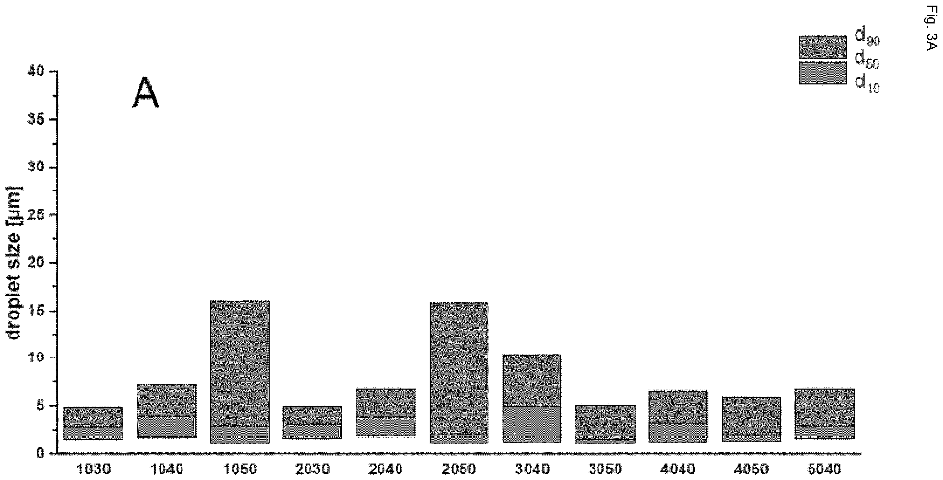

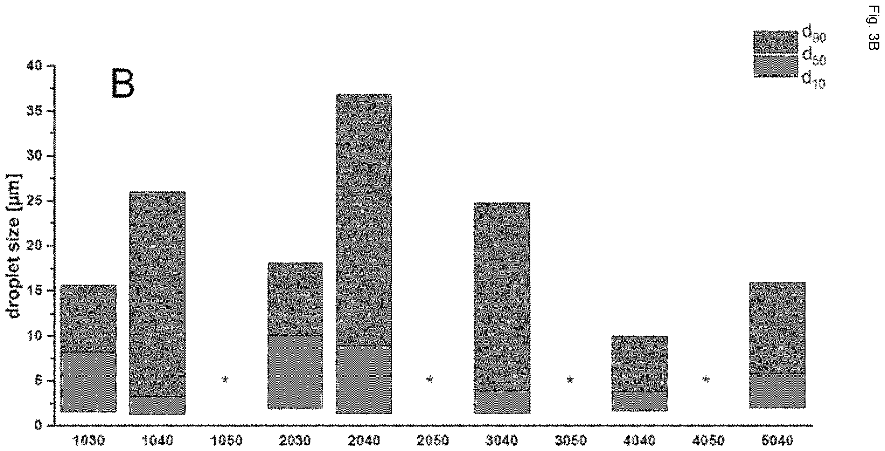

Furthermore, the droplet size was analysed in the formulations. The results are shown in Figs. 3A and 3B

Based on these results, six physically stable formulations (1030, 1040, 2030, 2040, 3040, 4040) were selected for stability studies after incorporation of sertaconazole nitrate.

### Example 10: Stability studies after incorporation of sertaconazole nitrate

The preferred formulations of Example 9 were prepared. Up to 1% (w/w) sertaconazole nitrate was added to the formulations. In cases with a saturation concentration below 1%, a concentration slightly below the saturation concentration was chosen, which resulted in the following compositions: 0.3S1030, 0.5S1040, 0.45S2030, 0.75S2040, 1S3040 and 1S4040.

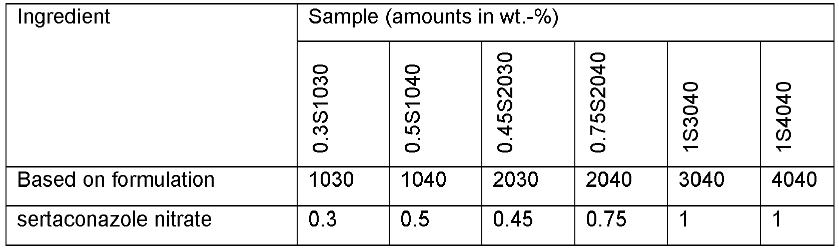

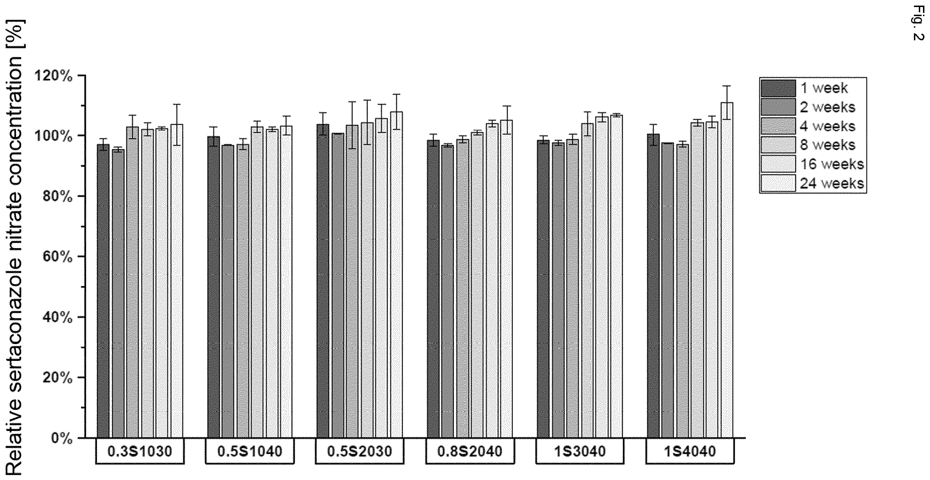

Sertaconazole nitrate did not change the consistencies and phase separation behaviour in comparison to the same formulations without sertaconazole nitrate. During storage at 30°C for 24 weeks, the microscopic appearance in a polarized light microscope was homogenous. None of the samples showed crystallization. In two cases, however, needle-shaped crystals were formed during storage at room temperature. This was the case in the formulations 2030 when 0.5 wt.-% sertaconazole nitrate was added and 2040 when 0.8 wt.-% sertaconazole nitrate was added. For further experiments, the concentration of sertaconazole nitrate was therefore reduced by 0.05 percentage points, which led to the compositions 0.45S2030 and 0.75S2040. The droplet size distribution after 24 weeks of storage was similar to the same formulations without sertaconazole nitrate, adding sertaconazole nitrate seems to have no influence on the physical stability of the systems. The content of sertaconazole nitrate was determined to rule out chemical instabilities of sertaconazole nitrate in combination with the ingredients of the formulations. Partial degradation of sertaconazole nitrate is generally tolerated if not more than 10% is lost during storage and if there are no toxic degradation products. The content of sertaconazole nitrate was not reduced over 24 weeks in all six formulations (Fig. 2), there were no signs of degradation.

### Example 11: Infected nail plate model - effect on surface

### Preparation of keratin films:

Keratin films and bovine hoof plates were prepared according to (Lusiana et al., European journal of pharmaceutics and biopharmaceutics 2011, 78 (3), 432-440) and (Täuber et al., Molecular pharmaceutics 2014, 11 (7), 1991-1996). Blonde or grey hair was washed, cut into pieces of 1-3 cm and dissolved in Shindai solution containing 25 mM Tris pH 8.5, 2.6 M thiourea, 5 M urea and 5% (v/v) 2-mercaptoethanol. After three days of stirring at 50°C and 100 rpm, the solution was filtered through four layers of medical gauze. The filtrate was centrifuged 2 times at 5,000 rpm for 30 minutes to remove undissolved remnants. Afterwards, the supernatant was filtered through a filter paper (pore size 2.5µm) using a water jet pump. 100 ml of the filtrate was given into a dialysis tube (Spectra/Por 1 Dialysis Membrane, MWCO 6-8 kDa) and dialyzed against water for 72 hours to remove remaining 2-mercaptoethanol. After that, the solution was centrifuged 3 times at 10,000 rpm for 30 minutes to remove aggregated particles. For making the keratin films, a PTFE ring made in our workshop was glued onto PET foil. Depending on the keratin content, 2-2.5 ml of dialysate were pipetted into the ring (added in two equal proportions spaced 6 hours apart) and were allowed to dry for at least 24 h at 40°C. After dry and clear films were obtained, they were cured for 3 hours at 110°C to oxidize disulfide bonds in the keratin structure. The thickness was determined using a micrometer screw gauge and ranged between 120 µm and 150 µm.

To obtain bovine hoof plates, pieces from dried bovine hooves (2x2 cm) were cut out. The pieces were kept in double distilled water for 1-3 days until they were soft enough to be cut. Slices of 120 µm thickness were cut with a rotational microtome MICROM HM 355S (Walldorf, Germany). The thickness was verified after hydration for one hour in PBS.

The infected nail plate model was set up according to (Lusiana et al., European journal of pharmaceutics and biopharmaceutics 2013, 84 (3), 599.605). Hoof plates or keratin films were autoclaved in PBS at 121°C for 15 minutes, inoculated with *Trichophyton rubrum* on potato glucose agar (PGA) and incubated for 7 days. The infected hoof plates or keratin films were then transferred onto Sabouraud dextrose agar (SDA). A PTFE ring was glued onto the nail model and a formulation was applied into the middle of the ring. The ring was then covered with PET foil to avoid evaporation. After 6 days, fungal growth on the border of the nail model was determined and a score was given. Scores ranged from 0 to 10, where 0 meant no fungal growth and 10 meant growth on the whole border. Scores in between were given for partial fungal growth, for example a score of 3 meant that 30% of the border were was affected. If there was fungal growth on the agar outside the border of the nail plate, a score of 0.5 was given.

In addition to poloxamer 407-based formulations, Mykosert^{®} Spray was used as a marketed comparator. Mykosert^{®} Spray is a liquid formulation containing 2% (w/v) sertaconazole nitrate and is registered used to treat skin infections with dermatophyte fungi, but not to treat onychomycosis.

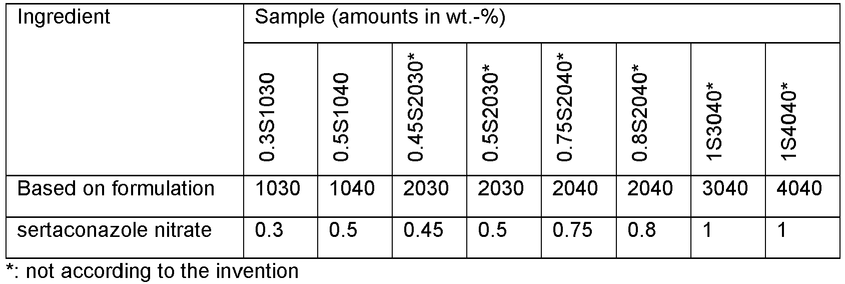

### Hoof plates:

| Formulation | N | Scores | Mean | Standard deviation |
|---|---|---|---|---|
| Blind | 10 | 10, 10, 10, 10, 10, 10, 10, 10, 10, 10 | 10 | 0 |
| 0.3S1030 | 8 | 2, 3, 4, 4, 4, 4, 6, 8 | 4.38 | 1.85 |
| 0.5S1040 | 7 | 0, 0, 0, 0, 0, 0, 0 | 0 | 0 |
| 0.5S2030* | 10 | 0, 0, 0, 0, 0, 0, 0, 0, 1, 5 | 0.6 | 1.58 |
| 0.75S2040* | 8 | 0, 0.5, 1, 1, 1, 3, 3, 3 | 1.56 | 1.24 |
| 0.8S2040* | 4 | 0, 0, 0, 0 | 0 | 0 |
| 1S3040* | 6 | 0, 0, 0, 0.5, 1, 3 | 0.75 | 1.17 |
| 1S4040* | 8 | 0, 1, 2.5, 3, 3, 3, 4, 5 | 2.69 | 1.58 |
| Mykosert | 9 | 2, 4, 5, 9, 9, 10, 10, 10, 10 | 7.67 | 3.12 |

| | | | | |
|---|---|---|---|---|
| *: not according to the invention | | | | |

### Keratin film:

| Formulation | N | Scores | Mean | Standard deviation |
|---|---|---|---|---|
| Blind | 6 | 10, 10, 10, 10, 10, 10 | 10 | 0 |
| 0.3S1030 | 4 | 8, 8, 10, 10 | 9 | 1.15 |
| 0.5S1040 | 6 | 0, 2, 2, 3, 4, 8 | 3.17 | 2.71 |
| 0.45S2030* | 4 | 6, 8, 8, 10 | 8 | 1.63 |
| 0.75S2040* | 4 | 2, 3, 3, 5 | 3.25 | 1.26 |
| 1S3040* | 4 | 1, 1, 3, 5 | 2.5 | 1.91 |
| 1S4040* | 4 | 0, 2, 3, 6 | 2.75 | 2.5 |
| Mykosert | 4 | 9, 9, 9, 9 | 9 | 0 |

| | | | | |
|---|---|---|---|---|
| *: not according to the invention | | | | |

An important difference between hoof plates and keratin film, which may be a reason for the different scores, is their ability to absorb water. Hoof plates can take up 45% of water relative to its dry weight, while keratin films only take up 5% (Lusiana et al., European journal of pharmaceutics and biopharmaceutics 2011, 78 (3), 432-440). It is to be noted that the literature describes an increasing discrepancy between hoof plates and keratin film permeability with increasing lipophilicity of the permeating molecule. This was thought to be caused by a specific binding of lipophilic substances by keratin films due to the different amino acid composition.

An effect on the fungal growth on the surface of the nail or keratin film could be observed for all of the tested formulations.

In the experiments with hoof plates, 0.5S2030 (including 0.5 wt.-% sertaconazole nitrate) was used, while in the experiments with KF the sertaconazole nitrate content was lowered resulting in the composition 0.45S2030 (with 0.45 wt.-% sertaconazole nitrate). Interestingly, in these two examples it can be observed that the additional 0.05 percentage points of sertaconazole nitrate result in a much better score compared to the other formulations. A supersaturated solution thus seems to be beneficial for penetration of sertaconazole nitrate into the nail plate model.

The high scores of the marketed comparator show that a composition optimized for application on the skin is not suitable for the treatment of fungal infections of the nail.

### Example 12: Permeation through keratin films

Keratin films were prepared as in Example 11. In vitro permeation studies were performed in modified Franz diffusion cells. The volume of the acceptor compartment ranged from 4.5ml to 7ml, the available area for the permeation from 0.39cm² to 0.57cm². The keratin films used as permeation barrier had a thickness of 120µm and were punched out to a diameter of 1.5 cm. Before the start of the experiment, they were hydrated in PBS for 60 minutes. Afterwards, the keratin films were fixed between the acceptor and donor compartment with silicone paste. The openings of the donor and the acceptor as well as the connecting area were sealed with Parafilm^{®} to prevent evaporation. Donor and acceptor were additionally fixed with a metal clamp. The Franz diffusion cells were stirred at 250 rpm and equilibrated at 32°C in a water bath to resemble the surface temperature of the skin. The acceptor medium consisted of 60% wt.-% PBS, 20% wt.-% IPA and 20% wt.-% PG. This composition was chosen to achieve a sufficient solubility of sertaconazole nitrate while resembling the composition of the P407-based formulations. Samples of 250 µl were taken after 1, 2, 4, 6, 8, 10, 21, 24, 27 and 30 hours and immediately replaced with fresh acceptor medium which was equilibrated at 32°C. The samples were quantified for sertaconazole nitrate contents via HPLC within 24 hours.

The formulations 0.3S1030, 0.5S1040 (both according to the invention), 0.45S2030, 0.75S2040, 1S3040 (each not according to the invention) and Mykosert^{®} Spray were tested.

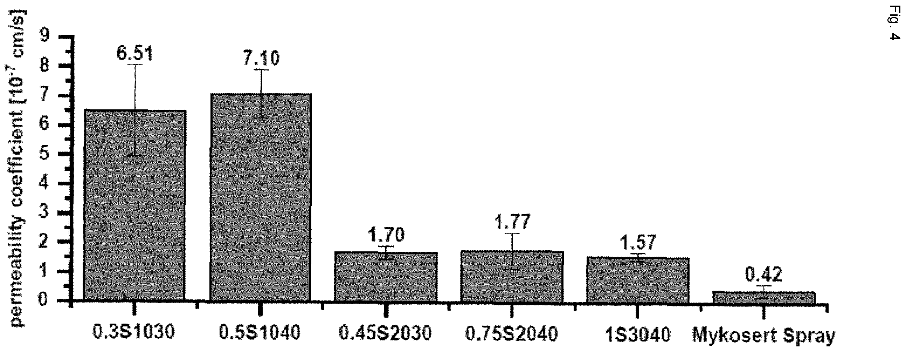

The permeated amount of sertaconazole nitrate over time is linear after a lag-time of approximately 4 hours for all tested formulations. The permeability coefficients of the poloxamer 407-based formulations, calculated between hour 4 and hour 30, ranged from 1.57×10-7cm/s to 7.10×10-7cm/s (Fig. 4). Surprisingly, the two formulations with the lowest amount of poloxamer 407, 0.3S1030 and 0.5S1040, have the highest permeability coefficient compared to the other poloxamer 407-based formulations. Mykosert^{®} Spray shows a lower permeability coefficient of 0.42×10-7cm/s.

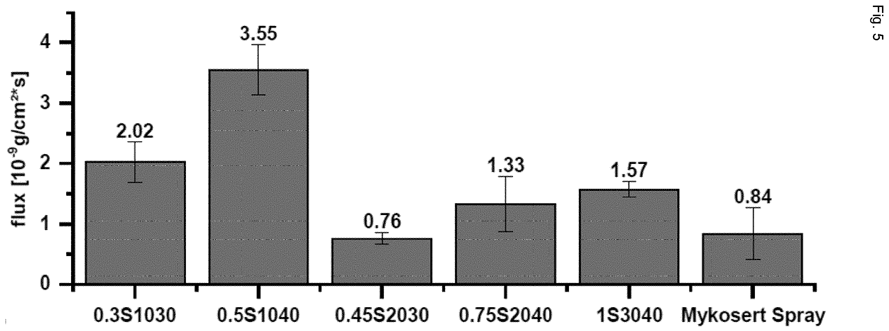

Comparing the actual flux of sertaconazole nitrate revealed that the formulations 0.3S1030 and 0.5S1040 provided the highest flux of sertaconazole nitrate, even though these formulations included low concentrations of sertaconazole nitrate (Fig. 5).

## Claims

1. Pharmaceutical composition comprising or consisting of
a) 0.1 to 2.5 wt.-%, related to the total weight of the composition, of sertaconazole or a salt thereof, preferably sertaconazole nitrate,
b) 60 to 80 wt.-%, related to the total weight of the composition, of one, two, three, four or more solvent(s), wherein the or one, two, three or four or, respectively, all solvent(s) is / are selected from the group consisting of isopropanol, ethanol, ethyl acetate and water,
c) 12.5 to 25 wt.-%, related to the total weight of the composition, of one, two, three, four or more solubilizer(s), wherein the or one, two, three, four or five or, respectively, all solubilizer(s) is/are selected from the group consisting of medium-chain triglycerides, propylene glycol, transcutol, polyglycol M350 and Macrogol 400 and
d) 5 to 12.5 wt.-%, related to the total weight of the composition, of one, two, three or more gel or film former(s), wherein the or one, two or three or, respectively, all gel or film former(s) is/are selected from the group consisting of poloxamer 407, Eudragit RS and Eudragit RL.

2. Pharmaceutical composition according to claim 1, wherein the composition comprises or consists of
a) 0.1 to 2.5 wt.-%, related to the total weight of the composition, of sertaconazole or a salt thereof, preferably sertaconazole nitrate,
b) 60 to 80 wt.-%, related to the total weight of the composition, of one, two, three, four or more solvent(s), wherein the or one, two, three or four or, respectively, all solvent(s) is / are selected from the group consisting of isopropanol, ethanol, ethyl acetate and water, wherein the total amount of the solvent(s) from the group consisting of isopropanol, ethanol, ethyl acetate and water in the composition is at least 60 wt.%,
c) 12.5 to 25 wt.-%, related to the total weight of the composition, of one, two, three, four or more solubilizer(s), wherein the or one, two, three, four or five or, respectively, all solubilizer(s) is/are selected from the group consisting of medium-chain triglycerides, propylene glycol, transcutol, polyglycol M350 and Macrogol 400, wherein the total amount of the solubilizer(s) from the group consisting of medium-chain triglycerides, propylene glycol, transcutol, polyglycol M350 and Macrogol 400 in the composition is at least 12.5 wt.%, and
d) 5 to 12.5 wt.-%, related to the total weight of the composition, of one, two, three or more gel or film former(s), wherein the or one, two or three or, respectively, all gel or film former(s) is/are selected from the group consisting of poloxamer 407, Eudragit RS and Eudragit RL, wherein the total amount of the gel or film former(s) from the group consisting of poloxamer 407, Eudragit RS and Eudragit RL in the composition is at least 5 wt.%.

3. Pharmaceutical composition according to any one of the preceding claims, wherein component b) comprises or consists of ethanol and ethyl acetate.

4. Pharmaceutical composition according to any of the preceding claims, wherein component d) comprises or consists of Eudragit RS or wherein component d) comprises or consists of Eudragit RL.

5. Pharmaceutical composition according to any of the preceding claims, wherein component a) is present in the range of from 1 to 2.5 wt.-%, related to the total weight of the composition,
and/or
component b) is present in the range of from 65 to 70 wt.-%, related to the total weight of the composition,
and/or
component c) is present in the range of from 17.5 to 22.5 wt.-%, related to the total weight of the composition,
and/or
component d) is present in the range of from 7.5 to 12.5 wt.-%, related to the total weight of the composition.

6. Pharmaceutical composition according to any one of claims 1, 2, 4 or 5, preferably according to any one of claims 1, 2 or 4, wherein component b) comprises or consists of isopropanol and water.

7. Pharmaceutical composition according to any one of claims 1 to 3, 5 or 6, preferably according to any one of claims 1 to 3 or 6, wherein component d) comprises or consists of poloxamer 407.

8. Pharmaceutical composition according to any one of the preceding claims, wherein component c) comprises or consists of propylene glycol.

9. Pharmaceutical composition according to any one of claims 1 to 8, wherein component c) consists of propylene glycol, transcutol, polyglykol M350 and macrogol 400.

10. Pharmaceutical composition according to any one of any one of claims 1 to 7, wherein component c) comprises or consists of propylene glycol and medium-chain triglycerides.

11. Pharmaceutical composition according to any one of claims 1 to 4 or 6 to 10, preferably according to any one of claims 1 to 2 or 6 to 8 or 10, wherein component a) is present in the range of from 0.1 to 0.75 wt.-%, related to the total weight of the composition,
and/or
component b) is present in the range of from 67.5 to 77.5 wt.-%, related to the total weight of the composition,
and/or
component c) is present in the range of from 15 to 25 wt.-%, related to the total weight of the composition,
and/or
component d) is present in the range of from 5 to 10 wt.-%, related to the total weight of the composition.

12. Pharmaceutical composition according to any one of the preceding claims for use in the treatment and/or prevention of nail disorders, preferably of nail mycosis, distal subungual onychomycosis, proximal subungual onychomycosis, white superficial onychomycosis, dystrophic onychomycosis and/or Candida-onychomycosis.

13. Medical kit, comprising a pharmaceutical composition according to any one of claims 1 to 11 and one or more additional components, preferably selected from the group consisting of files, application brushes, pipettes, containers, dispensers, pens, pads, tapes, foils, stickers, wraps, lamps, and polishes.

## Patentansprüche

1. Pharmazeutische Zusammensetzung umfassend oder bestehend aus
a) 0,1 bis 2,5 Gew.-%, bezogen auf das Gesamtgewicht der Zusammensetzung, an Sertaconazol oder einem Salz davon, vorzugsweise Sertaconazolnitrat,
b) 60 bis 80 Gew.-%, bezogen auf das Gesamtgewicht der Zusammensetzung, an einem, zwei, drei, vier oder mehr Lösungsmittel(n), wobei das oder ein, zwei, drei oder vier, bzw. alle Lösungsmittel ausgewählt ist/sind aus der Gruppe bestehend aus Isopropanol, Ethanol, Ethylazetat und Wasser,
c) 12,5 bis 25 Gew.-%, bezogen auf das Gesamtgewicht der Zusammensetzung, an einem, zwei, drei, vier oder mehr Lösungsvermittler(n), wobei der oder ein, zwei, drei, vier oder fünf, bzw. alle Lösungsvermittler ausgewählt ist/sind aus der Gruppe bestehend aus mittelkettigen Triglyceriden, Propylenglykol, Transcutol, Polyglykol M350 und Macrogol 400 und
d) 5 bis 12,5 Gew.-%, bezogen auf das Gesamtgewicht der Zusammensetzung, an einem, zwei, drei oder mehr Gel- oder Filmbildner(n), wobei der oder ein, zwei oder drei, bzw. alle Gel- oder Filmbildner ausgewählt ist/sind aus der Gruppe bestehend aus Poloxamer 407, Eudragit RS und Eudragit RL.

2. Pharmazeutische Zusammensetzung nach Anspruch 1, wobei die Zusammensetzung umfasst oder besteht aus
a) 0,1 bis 2,5 Gew.-%, bezogen auf das Gesamtgewicht der Zusammensetzung, an Sertaconazol oder einem Salz davon, vorzugsweise Sertaconazolnitrat,
b) 60 bis 80 Gew.-%, bezogen auf das Gesamtgewicht der Zusammensetzung, an einem, zwei, drei, vier oder mehr Lösungsmittel(n), wobei das oder ein, zwei, drei oder vier, bzw. alle Lösungsmittel ausgewählt ist/sind aus der Gruppe bestehend aus Isopropanol, Ethanol, Ethylazetat und Wasser, wobei die Gesamtmenge an Lösungsmittel(n) aus der Gruppe bestehend aus Isopropanol, Ethanol, Ethylazetat und Wasser in der Zusammensetzung mindestens 60 Gew.-% beträgt,
c) 12,5 bis 25 Gew.-%, bezogen auf das Gesamtgewicht der Zusammensetzung, an einem, zwei, drei, vier oder mehr Lösungsvermittler(n), wobei der oder ein, zwei, drei, vier oder fünf, bzw. alle Lösungsvermittler ausgewählt ist/sind aus der Gruppe bestehend aus mittelkettigen Triglyceriden, Propylenglykol, Transcutol, Polyglykol M350 und Macrogol 400, wobei die Gesamtmenge an Lösungsvermittler(n) aus der Gruppe bestehend aus mittelkettigen Triglyceriden, Propylenglykol, Transcutol, Polyglykol M350 und Macrogol 400 in der Zusammensetzung mindestens 12,5 Gew.-% beträgt, und
d) 5 bis 12,5 Gew.-%, bezogen auf das Gesamtgewicht der Zusammensetzung, an einem, zwei, drei oder mehr Gel- oder Filmbildner(n), wobei der oder ein, zwei oder drei, bzw. alle Gel- oder Filmbildner ausgewählt ist/sind aus der Gruppe bestehend aus Poloxamer 407, Eudragit RS und Eudragit RL, wobei die Gesamtmenge an Filmbildner(n) aus der Gruppe bestehend aus Poloxamer 407, Eudragit RS und Eudragit RL in der Zusammensetzung mindestens 5 Gew.-% beträgt.

3. Pharmazeutische Zusammensetzung nach einem der vorangehenden Ansprüche, wobei Komponente b) Ethanol und Ethylazetat umfasst oder daraus besteht.

4. Pharmazeutische Zusammensetzung nach einem der vorangehenden Ansprüche, wobei Komponente d) Eudragit RS umfasst oder daraus besteht, oder wobei Komponente d) Eudragit RL umfasst oder daraus besteht.

5. Pharmazeutische Zusammensetzung nach einem der vorangehenden Ansprüche, wobei Komponente a) in einem Bereich von 1 bis 2,5 Gew.-%, bezogen auf das Gesamtgewicht der Zusammensetzung, vorhanden ist,
und/oder
Komponente b) in einem Bereich von 65 bis 70 Gew.-%, bezogen auf das Gesamtgewicht der Zusammensetzung, vorhanden ist,
und/oder
Komponente c) in einem Bereich von 17,5 bis 22,5 Gew.-%, bezogen auf das Gesamtgewicht der Zusammensetzung, vorhanden ist,
und/oder
Komponente d) in einem Bereich von 7,5 bis 12,5 Gew.-%, bezogen auf das Gesamtgewicht der Zusammensetzung, vorhanden ist.

6. Pharmazeutische Zusammensetzung nach einem der Ansprüche 1, 2, 4 oder 5, vorzugsweise nach einem der Ansprüche 1, 2 oder 4, wobei Komponente b) Isopropanol und Wasser umfasst oder daraus besteht.

7. Pharmazeutische Zusammensetzung nach einem der Ansprüche 1 bis 3, 5 oder 6, vorzugsweise nach einem der Ansprüche 1 bis 3 oder 6, wobei Komponente d) Poloxamer 407 umfasst oder daraus besteht.

8. Pharmazeutische Zusammensetzung nach einem der vorangehenden Ansprüche, wobei Komponente c) Propylenglykol umfasst oder daraus besteht.

9. Pharmazeutische Zusammensetzung nach einem der Ansprüche 1 bis 8, wobei Komponente c) aus Propylenglykol, Transcutol, Polyglykol M350 und Macrogol 400 besteht.

10. Pharmazeutische Zusammensetzung nach einem der Ansprüche 1 bis 7, wobei Komponente c) Propylenglykol und mittelkettige Triglyceride umfasst oder daraus besteht.

11. Pharmazeutische Zusammensetzung nach einem der Ansprüche 1 bis 4 oder 6 bis 10, vorzugsweise nach einem der Ansprüche 1 bis 2 oder 6 bis 8 oder 10, wobei Komponente a) in einem Bereich von 0,1 bis 0,75 Gew.-%, bezogen auf das Gesamtgewicht der Zusammensetzung, vorhanden ist,
und/oder
Komponente b) in einem Bereich von 67,5 bis 77,5 Gew.-%, bezogen auf das Gesamtgewicht der Zusammensetzung, vorhanden ist,
und/oder
Komponente c) in einem Bereich von 15 bis 25 Gew.-%, bezogen auf das Gesamtgewicht der Zusammensetzung, vorhanden ist,
und/oder
Komponente d) in einem Bereich von 5 bis 10 Gew.-%, bezogen auf das Gesamtgewicht der Zusammensetzung, vorhanden ist.

12. Pharmazeutische Zusammensetzung nach einem der vorangehenden Ansprüche zur Verwendung in der Behandlung und/oder Prävention von Nagelerkrankungen, vorzugsweise Nagelmykose, distale subunguale Onychomykose, proximale subunguale Onychomykose, weiße oberflächliche Onychomykose, dystrophe Onychomykose und/oder Candida-Onychomykose.

13. Medizinisches Kit umfassend eine pharmazeutische Zusammensetzung nach einem der Ansprüche 1 bis 11 und eine oder mehrere zusätzliche Komponente(n), vorzugsweise ausgewählt aus der Gruppe bestehend aus Feilen, Auftragspinseln, Pipetten, Behältern, Dispensern, Stiften, Pads, Bändern, Folien, Aufklebern, Verpackungen, Lampen und Polituren.

## Revendications

1. Composition pharmaceutique comprenant ou consistant en
a) 0,1 à 2,5 % en poids, par rapport au poids total de la composition, de sertaconazole ou d'un de ses sels, de préférence de nitrate de sertaconazole,
b) 60 à 80 % en poids, par rapport au poids total de la composition, d'un, de deux, trois, quatre solvant(s) ou plus, dans laquelle le ou un, deux, trois ou quatre ou, respectivement, tous les solvants est/sont choisi(s) dans le groupe constitué par l'isopropanol, l'éthanol, l'acétate d'éthyle et l'eau,
c) 12,5 à 25 % en poids, par rapport au poids total de la composition, d'un, de deux, trois, quatre solubilisant(s) ou plus, dans laquelle le ou un, deux, trois, quatre ou cinq ou, respectivement, tous les solubilisants est/sont choisi(s) dans le groupe constitué par les triglycérides à chaîne moyenne, le propylène glycol, le transcutol, le polyglycol M350 et le Macrogol 400 et
d) 5 à 12,5 % en poids, par rapport au poids total de la composition, d'un, de deux, trois d'agent(s) gélifiant(s) ou filmogène(s) ou plus, dans laquelle le ou un, deux ou trois ou, respectivement, tous les agents gélifiants ou filmogènes est/sont choisi(s) dans le groupe consistant en poloxamère 407, Eudragit RS et Eudragit RL.

2. Composition pharmaceutique selon la revendication 1, dans laquelle la composition comprend ou consiste en
a) 0,1 à 2,5 % en poids, par rapport au poids total de la composition, de sertaconazole ou d'un de ses sels, de préférence de nitrate de sertaconazole,
b) 60 à 80 % en poids, par rapport au poids total de la composition, d'un, de deux, trois, quatre solvant(s) ou plus, dans lequel le ou un, deux, trois ou quatre ou, respectivement, tous les solvants est/sont choisi(s) dans le groupe constitué par l'isopropanol, l'éthanol, l'acétate d'éthyle et l'eau, dans laquelle la quantité totale de solvant(s) du groupe constitué par l'isopropanol, l'éthanol, l'acétate d'éthyle et l'eau dans la composition est d'au moins 60 % en poids,
c) 12,5 à 25 % en poids, par rapport au poids total de la composition, d'un, de deux, trois, quatre solubilisant(s) ou plus, dans lequel le ou un, deux, trois, quatre ou cinq ou, respectivement, tous les solubilisants est/sont choisi(s) dans le groupe constitué par les triglycérides à chaîne moyenne, le propylène glycol, le transcutol, le polyglycol M350 et le Macrogol 400, dans laquelle la quantité totale de solubilisant(s) dans le groupe constitué par les triglycérides à chaîne moyenne, le propylène glycol, le transcutol, le polyglycol M350 et le Macrogol 400 dans la composition est d'au moins 12,5 % en poids, et
d) 5 à 12,5 % en poids, par rapport au poids total de la composition, d'un, de deux, trois d'agent(s) gélifiant(s) ou filmogène(s) ou plus, dans laquelle le ou un, deux ou trois ou, respectivement, tous les agents gélifiants ou filmogènes est/sont choisi(s) dans le groupe consistant en le poloxamère 407, l'Eudragit RS et l'Eudragit RL, dans laquelle la quantité totale de l' (des) agent(s) gélifiant(s) ou filmogène(s) dans le groupe consistant en le poloxamère 407, l'Eudragit RS et l'Eudragit RL dans la composition est d'au moins 5 % en poids.

3. Composition pharmaceutique selon l'une quelconque des revendications précédentes, dans laquelle le composant b) comprend le ou consiste en éthanol et acétate d'éthyle.

4. Composition pharmaceutique selon l'une quelconque des revendications précédentes, dans laquelle le composant d) comprend le ou consiste en Eudragit RS ou dans laquelle le composant d) comprend le ou consiste en Eudragit RL.

5. Composition pharmaceutique selon l'une quelconque des revendications précédentes, dans laquelle le composant a) est présent dans la plage allant de 1 à 2,5 % en poids, par rapport au poids total de la composition,
et/ou
le composant b) est présent dans la plage allant de 65 à 70 % en poids, par rapport au poids total de la composition,
et/ou
le composant c) est présent dans la plage allant de 17,5 à 22,5 % en poids, par rapport au poids total de la composition,
et/ou
le composant d) est présent dans la plage allant de 7,5 à 12,5 % en poids, par rapport au poids total de la composition.

6. Composition pharmaceutique selon l'une quelconque des revendications 1, 2, 4 ou 5, de préférence selon l'une quelconque des revendications 1, 2 ou 4, dans laquelle le composant b) comprend l'isopropanol et l'eau ou est constitué de ceux-ci.

7. Composition pharmaceutique selon l'une quelconque des revendications 1 à 3, 5 ou 6, de préférence selon l'une quelconque des revendications 1 à 3 ou 6, dans laquelle le composant d) comprend le ou est constitué de poloxamère 407.

8. Composition pharmaceutique selon l'une quelconque des revendications précédentes, dans laquelle le composant c) comprend le ou est constitué de propylène glycol.

9. Composition pharmaceutique selon l'une quelconque des revendications 1 à 8, dans laquelle le composant c) est constitué de propylène glycol, de transcutol, de polyglycol M350 et de Macrogol 400.

10. Composition pharmaceutique selon l'une quelconque des revendications 1 à 7, dans laquelle le composant c) comprend le propylène glycol et les triglycérides à chaîne moyenne ou est constitué de ceux-ci.

11. Composition pharmaceutique selon l'une quelconque des revendications 1 à 4 ou 6 à 10, de préférence selon l'une quelconque des revendications 1 à 2 ou 6 à 8 ou 10, dans laquelle le composant a) est présent dans la plage allant de 0,1 à 0,75 % en poids, par rapport au poids total de la composition,
et/ou
le composant b) est présent dans la plage allant de 67,5 à 77,5 % en poids, par rapport au poids total de la composition,
et/ou
le composant c) est présent dans la plage allant de 15 à 25 % en poids, par rapport au poids total de la composition,
et/ou
le composant d) est présent dans la plage allant de 5 à 10 % en poids, par rapport au poids total de la composition.

12. Composition pharmaceutique selon l'une quelconque des revendications précédentes, pour l'utilisation dans le traitement et/ou la prévention des affections unguéales, de préférence de la mycose unguéale, de l'onychomycose sous-unguéale distale, de l'onychomycose sous-unguéale proximale, de l'onychomycose superficielle blanche, de l'onychomycose dystrophique et/ou de l'onychomycose à Candida.

13. Kit médical comprenant une composition pharmaceutique selon l'une quelconque des revendications 1 à 11 et un ou plusieurs composants additionnels, de préférence choisis dans le groupe constitué par les limes, les pinceaux d'application, les pipettes, les récipients, les distributeurs, les stylos, les tampons, les rubans, les feuilles, les autocollants, les emballages, les lampes et les polis.
